# EUROPEAN PATENT APPLICATION

(11) **EP 3 243 505 A1**
(43) Date of publication of application: **15.11.2017**
(21) Application number: 16460027.2
(22) Date of filing: 13.05.2016
(51) Int. Cl.: A61K 9/14, A61K 9/16, A61K 9/20

(54) **A PHARMACEUTICAL COMPOSITION COMPRISING AMORPHOUS APIXABAN**

(71) Applicant: Zaklady Farmaceutyczne Polpharma SA, 83-200 Starogard Gdanski (PL)
(72) Inventor: Garbera, Kamil, 59-220 Legnica (PL); Kislo, Kamil, 19-300 Elk (PL); Cichocki, Marek, 31-416 Krakow (PL)

(57) **Abstract**

The present invention relates to a pharmaceutical composition comprising a therapeutically effective amount of apixaban or a salt thereof together with one or more pharmaceutically acceptable excipients or carriers, wherein apixaban is amorphous and the composition exhibits an in vitro dissolution profile measured in phosphate buffer at pH 6.8 containing 0.05 % weight/volume of sodium lauryl sulfate according to which: at least 30% of apixaban is dissolved after 5 minutes and at least 60% of apixaban is dissolved after 10 minutes; processes for its preparation and its use for the treatment of thromboembolic diseases or disorders.

## Description

### FIELD OF THE INVENTION

The present invention relates to a pharmaceutical composition comprising amorphous apixaban having a particular in vitro dissolution profile, their preparations processes and their use for the treatment of a venous thromboembolic disease or disorder.

### BACKGROUND ART

Apixaban is the International Nonproprietary Name (INN) of
1-(4-methoxyphenyl)-7-oxo-6-[4-(2-oxopiperidin-1-yl)phenyl]-4,5,6,7-tetrahydro-1H-pyrazolo[3,4-c]pyridine-3-carboxamide. Apixaban is a highly selective, orally bioavailable, and reversible direct inhibitor of free and clot-bound factor Xa. Factor Xa catalysis the conversion of prothrombin to thrombin, the final enzyme in the coagulation cascade that is responsible for fibrin clot formation. Apixaban has no direct effect on platelet aggregation, but by inhibiting factor Xa, it indirectly decreases clot formation induced by thrombin, and thereof is used as anticoagulant for the treatment of venous thromboembolic events.

The structure of Apixaban corresponds to formula (I):

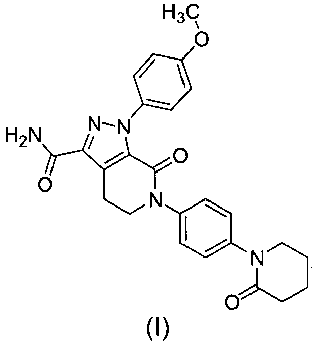

It was firstly disclosed in US patent 6967208 and it is currently marketed under the name of Eliquis® for the treatment and prophylaxis of deep vein thrombosis (DVT) and pulmonary embolism (PE). Eliquis exhibits rapid disintegration and dissolution characteristics.

Apixaban is known as a non-ionisable compound that has been classified as a high solubility/low permeability compound (Class III of the Biopharmaceutical Classification System -BCS). It is also known that apixaban is highly soluble for doses up to 10 mg and is a low permeable compound since the fraction of oral dose absorbed is less than 90%. Thus, the above mentioned dose of apixaban and its dissolution properties result in a slow and incomplete drug release and a poor availability.

In this regard, the PCT patent application WO2011106478 discloses that compositions of apixaban prepared by dry granulation processes have more consistent dissolution rates than those compositions prepared by a wet granulation processes which compromise the bioavailability of the active ingredient. Further, the PCT patent application WO2011106478 identifies the particle size of apixaban as a critical factor to modify the bioavailability of apixaban due to its influence on the dissolution of the tablets. In particular, the PCT patent application WO2011106478 discloses a composition which comprises crystalline particles of apixaban having a maximally limited mean particle size which is equal to or less than about 89 µm. These compositions allow achieving the appropriate rapid dissolution profile for being used as anticoagulant agent. Unfortunately, the use of a crystalline form of apixaban having a specific particle size requires additional steps such as milling or micronization of the active ingredients that hinder the process.

The PCT patent application WO2015121472 discloses a composition which comprises apixaban and a polymer having low viscosity as a wet granulation binder. However, these compositions already exhibit a poor dissolution and bioavailability in comparison with the commercially available Eliquis®.

From what is known in the art it is derived that there is still the need of providing pharmaceutical compositions of apixaban having the target dissolution profile of the marketed Eliquis®. The target dissolution profile requires that at least 30% of apixaban is dissolved after 5 minutes and at least 60% of apixaban is dissolved after 10 minutes when measured in phosphate buffer at pH 6.8 containing 0.05 % weight/volume of sodium lauryl sulfate.

### SUMMARY OF THE INVENTION

Inventors have found that a pharmaceutical composition comprising amorphous apixaban has an appropriate dissolution profile and shows a good bioavailability of the active ingredient. In particular, the compositions of the invention exhibits an in vitro dissolution profile measured in phosphate buffer at pH 6.8 containing 0.05 % weight/volume of sodium lauryl sulfate according to which: at least 30% of apixaban is dissolved after 5 minutes and at least 60% of apixaban is dissolved after 10 minutes. Thus, they exhibit the same dissolution profile as the marketed product Eliquis which comprises crystalline apixaban.

The compositions of the invention which comprises amorphous apixaban are also advantageous because they can be manufactured by simple, fast and industrial scale-up processes unlike the processes of prior art compositions which require concrete and laborious process conditions for reducing and controlling the particle size of the active ingredient.

Additionally, the process of the invention also allows using as a starting material apixaban in both amorphous form and crystalline form. When crystalline apixaban is used as a starting material, there is no need to control the apixaban particle size.

Accordingly, an aspect of the present invention refers to a pharmaceutical composition comprising a therapeutically effective amount of apixaban or a salt thereof together with one or more pharmaceutically acceptable excipients or carriers, wherein apixaban is amorphous and the composition exhibits an in vitro dissolution profile measured in phosphate buffer at pH 6.8 containing 0.05 % weight/volume of sodium lauryl sulfate according to which: at least 30% of apixaban is dissolved after 5 minutes and at least 60% of apixaban is dissolved after 10 minutes.

The second aspect of the present invention refers to a wet granulation tablet comprising a therapeutically effective amount of apixaban or a salt thereof together with one or more pharmaceutically acceptable excipients or carriers, wherein apixaban is amorphous and the composition exhibits an in vitro dissolution profile measured in phosphate buffer at pH 6.8 containing 0.05 % weight/volume of sodium lauryl sulfate according to which: at least 30% of apixaban is dissolved after 5 minutes and at least 60% of apixaban is dissolved after 10 minutes.

The third aspect of the present invention refers to a hot-melt extruded tablet comprising a therapeutically effective amount of apixaban or a salt thereof together with one or more pharmaceutically acceptable excipients or carriers, wherein apixaban is amorphous and the composition exhibits an in vitro dissolution profile measured in phosphate buffer at pH 6.8 containing 0.05 % weight/volume of sodium lauryl sulfate according to which: at least 30% of apixaban is dissolved after 5 minutes and at least 60% of apixaban is dissolved after 10 minutes.

The fourth aspect of the present invention refers to a process for the preparation of the wet granulation composition as defined in the second aspect of the invention, which comprises: (a) preparing a granulation dispersion of apixaban by: (i) heating a mixture selected from the group consisting of C₁-C₄ ketone and water; C₁-C₄ alcohol and water; C₁-C₄ ketone and C₁-C₄ alcohol; and C₁-C₄ ketone, C₁-C₄ alcohol, and water at a temperature from 50 to 70°C; (ii) adding the surfactant and optionally the binder in the solution obtained in step (i); and (iii) adding amorphous apixaban, or alternatively crystalline apixaban, or alternatively a mixture of amorphous and crystalline apixaban in the solution obtained in step (ii) at a temperature from 50°C to 70°C; and (b) applying the granulation dispersion of apixaban obtained in step (a) onto a filler by spray drying; or alternatively (a) preparing a granulation dispersion of apixaban by: (i) heating a mixture selected from the group consisting of C₁-C₄ ketone and water; C₁-C₄ alcohol and water; C₁-C₄ ketone and C₁-C₄ alcohol; and C₁-C₄ ketone, C₁-C₄ alcohol, and water at a temperature from 50°C to 70°C; (ii) adding amorphous apixaban, or alternatively crystalline apixaban, or alternatively a mixture of amorphous and crystalline apixaban in the solution obtained in step (i) at a temperature from 50°C to 70°C; and (iii) adding the surfactant and optionally the binder in the solution obtained in step (ii); and (b) applying the granulation dispersion of apixaban obtained in step (a) onto a filler by spray drying.

The fifth aspect of the present invention refers to a process for the preparation of a hot-melt extruded tablet as defined in the third aspect of the invention, which comprises: (i) mixing amorphous apixaban, or alternatively crystalline apixaban, or alternatively a mixture amorphous and crystalline apixaban with the hot-melt extrudable polymer and the hot-melt extrudable plasticizer; (ii) extruding the mixture obtained in step (i) by hot melt extrusion to obtain an extrudate; (iii) milling the extrudate obtained in step (ii); (iv) mixing the filler, the disintegrant and the lubricant to obtain a mixture A; (v) mixing the mixture A obtained in step (iv) with the extrudate obtained in step (ii); (vi) compressing the mixture obtained in step (v) into tablets; and optionally, (vii) film coating the tablets obtained in step (vi).

Finally, the sixth aspect of the present invention refers to a pharmaceutical composition as defined in the first aspect of the invention, for use in the treatment of venous thrombotic disease or disorder.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

For the purposes of the invention, any ranges given include both the lower and the upper end-points of the range. Ranges given, such as temperatures, times, and the like, should be considered approximate, unless specifically stated.

The expressions "obtainable", "obtained" and equivalent expressions are used interchangeably, and in any case, the expression "obtainable" encompasses the expression "obtained".

The term "therapeutically effective amount" of apixaban refers to that amount which provides the therapeutic effect after its application, which is the treatment of venous thrombotic disease or condition.

The term apixaban compound encompasses apixaban, as well as any pharmaceutically acceptable salt thereof.

The term "pharmaceutically acceptable" refers to that excipients or carriers suitable for use in the pharmaceutical technology for preparing compositions with medical use.

The terms "particle size distribution" or "PSD" have the same meaning and are used interchangeably. They refer to the percentage of the active ingredient used as a starting material (amorphous apixaban, crystalline apixaban or a mixture of amorphous apixaban and crystalline apixaban) within a certain size range. This term is expressed as Dx wherein X% of the volume of particles have a diameter less than a specified diameter D. For example, the term "D90" refers to the value of particle size distribution where at least 90% of the apixaban have a size less or equal to the given value. Further, the term "D50" refers to the value of particle size distribution where at least 50% of the particles have a size less or equal to the given value. The measurement was performed with an appropriate apparatus by conventional analytical techniques. In the present invention the particle size distribution was analysed by laser diffraction spectroscopy, G3 Morphology, and Raman spectroscopy as it is disclosed in section of general considerations of the Examples.

The term "percentage (%) by weight" refers to the percentage of each ingredient of the pharmaceutical composition in relation to the total weight of the composition.

The term "weight ratio" refers to the relation of weight of the solvents of the granulation dispersion needed to prepare the granulation dispersion.

The term "binder" refers any pharmaceutically acceptable compound having binding properties. Materials commonly used as binders include povidone such as polyvinylpyrrolidone K25, methylcellulose polymers, hydroxyethyl cellulose, hydroxypropyl cellulose, L-hydroxypropyly cellulose (low substituted), hydroxypropylmethyl cellulose (HPMC), sodium carboxymethyl cellulose, carboxymethylene, carboxymethylhydroxyethyl cellulose and other cellulose derivatives, starches or modified starches and mixture thereof.

The terms "filler" and "diluent" have the same meaning and are used interchangeably. They refer to any pharmaceutically acceptable filler or diluent that fill out the size of a composition, making it practical to produce and convenient for the consumer to use. Carrier is a base for API, which is physically connected to the surface of the carrier. Materials commonly used as filler include calcium carbonate, calcium phosphate, dibasic calcium phosphate, tribasic calcium sulfate, calcium carboxymethyl cellulose, cellulose, cellulose products such as microcrystalline cellulose and its salts, dextrin derivatives, dextrin, dextrose, fructose, lactitol, lactose, starches or modified starches, magnesium carbonate, magnesium oxide, maltitol, maltodextrins, maltose, mannitol, sorbitol, starch, sucrose, sugar, xylitol, erythritol and mixtures thereof.

The term "surfactant" refers to a compound that lowers the surface tension between two liquids or between a liquid and a solid. Materials commonly used as a surfactant include to: (a) nonionic surfactants such as polyoxyethylene fatty alcohol ethers, sorbitan fatty acid esters, polyoxyethylen fatty acid esters, sorbitan esters, glycerol monostearate, polyethylene glycols, cetyl alcohol, cetostearyl alcohol, stearyl alcohol and poloxamers; (b) anionic surfactants such as potassium laureate, triethanolamine stearate, sodium lauryl sulfate and alkyl polyoxyethylene sulfates; and (c) cationic surfactants such as quaternary ammonium compounds, benzalkonium chloride, cetyltrimethylammonium bromide and chitosans; and mixtures thereof.

The term "disintegrant" refers to a substance which helps the composition break up once ingested. Materials commonly used as a disintegrant are, but not limited to, cross linked polyvinylpyrolidone; starches such as maize starch and dried sodium starch glycolate; gums such as maize starch and dried sodium starch glycolate; gums such as alginic acid, sodium alginate, guar gum; croscarmellose sodium; low-substituted hydroxypropyl cellulose and mixtures thereof.

The term "glidant" refers to a substance which improves the flow characteristics of powder mixtures in the dry state. Materials commonly used as a glidant include magnesium stearate, colloidal silicon dioxide or talc.

The term "lubricant" refers to a substance that prevents composition ingredients from clumping together and from sticking to the tablet punches or capsule filling machine and improves flowability of the composition mixture. Materials commonly used as a lubricant include sodium oleate, sodium stearate, sodium benzoate, sodium stearate, sodium chloride, stearic acid, sodium stearyl fumarate, calcium stearate, magnesium stearate, magnesium lauryl sulfate, sodium stearyl fumarate, sucrose esters or fatty acid, zinc, polyethylene glycol, talc and mixtures thereof. The presence of a lubricant is particularly preferred when the composition is a tablet to improve the tableting process.

The term "ketone" refers to a molecule containing a carbonyl functional group bridging two groups of atoms and relates to compounds of the general formula R₂C(=O) being R (C₁-C₄) alkyl. Examples of ketones appropriate for the present invention include acetone, methyl ethyl ketone, methyl propyl ketone, diethyl ketone, methyl butyl ketone, methyl isobutyl ketone, mesityl oxide, cyclopentanone, cyclohexanone, acetophenone, propiophenone and benzophenone.

The term "alkyl" refers to a linear or branched hydrocarbon chain which contains the number of carbon atoms specified in the description or the claims. Preferably, the alkyl group is selected from methyl, ethyl and isopropyl.

The term "alcohol" refers to a hydrocarbon derivative having the number of atoms specific in the description and claims in which one or more hydrogen atoms have been replaced by one or more -OH group. The term alcohol also includes glycol compounds. Examples of alcohols appropriate for the present invention include ethanol, 2-propanol (isopropanol) and n-propanol.

As mentioned above, an aspect of the present invention refers to a pharmaceutical composition comprising a therapeutically effective amount of apixaban or a salt thereof together with one or more pharmaceutically acceptable excipients or carriers, wherein apixaban is amorphous and the composition exhibits an in vitro dissolution profile measured in phosphate buffer at pH 6.8 containing 0.05% weight/volume of sodium lauryl sulfate according to which: at least 30% of apixaban is dissolved after 5 minutes and at least 60% of apixaban is dissolved after 10 minutes.

For the purposes of the invention, the term "amorphous" refers to the active ingredient apixaban that shows at the X-ray diffraction pattern a large bump (or halo) distributed in a wide range (2 Theta).

In an embodiment, optionally in combination with one or more features of the various embodiments described above or below, the composition of the invention exhibits an in vitro dissolution profile measured in phosphate buffer at pH 6.8 containing 0.05% weight/volume of sodium lauryl sulfate according to which: at least 35% of apixaban is dissolved after 5 minutes and at least 65% of apixaban is dissolved after 10 minutes.

In an embodiment, optionally in combination with one or more features of the various embodiments described above or below, the composition of the invention exhibits an in vitro dissolution profile measured in phosphate buffer at pH 6.8 containing 0.05% weight/volume of sodium lauryl sulfate according to which: at least 40% of apixaban is dissolved after 5 minutes and at least 70% of apixaban is dissolved after 10 minutes.

In an embodiment, optionally in combination with one or more features of the various embodiments described above or below, the composition of the invention exhibits an in vitro dissolution profile measured in phosphate buffer at pH 6.8 containing 0.05% weight/volume of sodium lauryl sulfate according to which: at least 42% of apixaban is dissolved after 5 minutes and at least 74% of apixaban is dissolved after 10 minutes.

In an embodiment, optionally in combination with one or more features of the various embodiments described above or below, the composition of the invention exhibits an in vitro dissolution profile measured in phosphate buffer at pH 6.8 containing 0.05% weight/volume of sodium lauryl sulfate according to which: at least 44% of apixaban is dissolved after 5 minutes and at least 80% of apixaban is dissolved after 10 minutes.

As it is illustrated in the examples, the compositions of the invention as defined above are advantageous because they have a comparable dissolution profile to the commercially available composition of apixaban crystalline Eliquis.

In an embodiment, optionally in combination with one or more features of the various embodiments described above or below, the therapeutically effective amount of apixaban or a salt thereof is from 1% to 10% by weight; preferably from 1% to 5% by weight. In a particular embodiment, optionally in combination with one or more features of the various embodiments described above or below, the therapeutically effective amount of apixaban or a salt thereof is 2.5% by weight.

The compositions of the invention can be formulated in any form that include any unit dosage form, particularly any solid unit dosage form such as tablets, capsules, granules and powder; preferably, the composition is in form of tablets.

The compositions of the present invention can be prepared according to methods well known in the state of the art. The appropriate excipients and/or carriers, and their amounts, can readily be determined by those skilled in the art according to the type of formulation being prepared.

In an embodiment, optionally in combination with one or more features of the various embodiments described above or below, the composition of the invention is a wet granulation tablet; or a hot-melt extruded tablet.

In an embodiment, optionally in combination with one or more features of the various embodiments described above or below, the composition of the invention is a wet granulation tablet. In an embodiment, optionally in combination with one or more features of the various embodiments described above or below, the composition is a wet granulation tablet, and comprises: from 1-10% by weight of amorphous apixaban; from 1-12% by weight of surfactant; from 0-20% by weight of binder; from 35-95% by weight of filler; from 1-20% by weight of disintegrant; and from 1-5% by weight of lubricant, being the sum of the ingredients 100%.

In an embodiment, optionally in combination with one or more features of the various embodiments described above or below, the composition is a wet granulation tablet and the composition is that wherein the filler is lactose; preferably lactose monohydrate. In an embodiment, optionally in combination with one or more features of the various embodiments described above or below, the composition is a wet granulation tablet and the filler is in an amount from 35 to 95% by weight; preferably from 60 to 90% by weight. In an embodiment, optionally in combination with one or more features of the various embodiments described above or below, the composition is a wet granulation tablet and the filler is lactose monohydrate in an amount from 70% to 85% by weight.

In an embodiment, optionally in combination with one or more features of the various embodiments described above or below, the composition is a wet granulation tablet and the composition is that wherein the surfactant is selected from the group consisting of sodium laurilsulfate, polyoxyethylene-polypropylene block copolymer (poloxamer), polyoxyethylene sorbitan fatty acid esters, triethyl citrate and mixture thereof; preferably sodium laurilsulfate and polyoxyethylene-polypropylene block copolymer (poloxamer). The amount of the surfactant can be from 1% to 10% by weight; preferably from 1% to 5% by weight. In an embodiment, optionally in combination with one or more features of the various embodiments described above or below, the composition is a wet granulation tablet and the composition is that wherein the surfactant is sodium laurilsulfate and is in an amount from 1% to 3% by weight. In an embodiment, optionally in combination with one or more features of the various embodiments described above or below, the composition is a wet granulation tablet and the composition is that wherein the surfactant is also polyoxyethylene-polypropylene block copolymer (poloxamer) and is in an amount from 8% to 10% by weight; preferably 10% by weight.

In an embodiment, optionally in combination with one or more features of the various embodiments described above or below, the composition is a wet granulation tablet and the composition is that wherein the binder is selected from the group consisting of polyvinylpyrrolidone, hydroxypropyl cellulose, hydroxypropylmethyl cellulose, polyvinyl alcohol-polyethylen glycol graft copolymer, and mixture thereof; preferably polyvinylpyrrolidone; more preferably polyvinylpyrrolidone K25. The amount of the binder can be from 0% to 20% by weight; preferably from 0% to 15% by weight; more preferably 10% by weight. In an embodiment, optionally in combination with one or more features of the various embodiments described above or below, the composition is a wet granulation tablet and the composition is that wherein the binder is polyvinylpyrrolidone in an amount from 0% to 15% by weight; preferably 10% by weight.

In an embodiment, optionally in combination with one or more features of the various embodiments described above or below, the composition is a wet granulation tablet and the composition is that wherein the disintegrant is selected from the group consisting of croscarmellose sodium, crospovidone, sodium starch glycolate, polacrilin potassium, and mixture thereof; preferably croscarmellose sodium. The amount of the disintegrant can be from 1% to 20% by weight; preferably from 2% to 10% by weight; more preferably 5% by weight. In an embodiment, optionally in combination with one or more features of the various embodiments described above or below, the composition is a wet granulation tablet and the composition is that wherein the disintegrant is croscarmellose sodium in an amount from 2% to 10% by weight; preferably 5% by weight.

In an embodiment, optionally in combination with one or more features of the various embodiments described above or below, the composition is a wet granulation tablet and the composition is that wherein the lubricant is selected from the group consisting of magnesium stearate, sodium stearyl fumarate, glyceryl behenate, and mixture thereof; preferably magnesium stearate. The amount of the lubricant can be from 1 % to 5% by weight; preferably from 1 % to 3% by weight. In an embodiment, optionally in combination with one or more features of the various embodiments described above or below, the composition is a wet granulation tablet and the composition is that wherein the lubricant is magnesium stearate in an amount from 1% to 3% by weight.

In an embodiment, optionally in combination with one or more features of the various embodiments described above or below, the composition is a wet granulation tablet and the composition comprises: from 1-10% by weight of amorphous apixaban; from 1-10% by weight of a surfactant selected from sodium laurilsulfate, polyoxyethylene-polypropylene block copolymer and mixture thereof; from 1-20% by weight of polyvinylpyrrolidone; from 35-95% by weight of lactose monohydrate; from 1-20% by weight of croscarmellose sodium; and from 1-5% by weight of magnesium stearate, being the sum of the ingredients 100%.

In an embodiment, optionally in combination with one or more features of the various embodiments described above or below, the composition is a wet granulation tablet and the composition comprises: from 1-10% by weight of amorphous apixaban; from 1-12% by weight of a surfactant selected from sodium laurilsulfate, polyoxyethylene-polypropylene block copolymer and mixtures thereof; from 35-95% by weight of lactose monohydrate; from 1-20% by weight of croscarmellose sodium; and from 1-5% by weight of magnesium stearate, being the sum of the ingredients 100%.

In an embodiment, optionally in combination with one or more features of the various embodiments described above or below, the composition is a wet granulation tablet and the composition comprises: 2.5% by weight of amorphous apixaban; 10.0% by weight of polyvinylpyrrolidone; 71.50% by weight of lactose monohydrate; 10.0% by weight of a polyoxyethylene-polyoxypropylene block copolymer; 5.0% by weight of croscarmellose sodium; and 1.0% by weight of magnesium stearate.

In an embodiment, optionally in combination with one or more features of the various embodiments described above or below, the composition is a wet granulation tablet and the composition comprises: 2.5% by weight of amorphous apixaban; 1.25% by weight of sodium laurilsulfate; 80.25% by weight of lactose monohydrate; 10.0% by weight of a polyoxyethylene-polyoxypropylene block copolymer; 5.0% by weight of croscarmellose sodium; and 1.0% by weight of magnesium stearate.

In an embodiment, optionally in combination with one or more features of the various embodiments described above or below, the composition is a wet granulation tablet and the composition comprises: 2.5% by weight of amorphous apixaban; 1.25% by weight of sodium laurilsulfate;
10.0% by weight of polyvinylpyrrolidone; 80.25% by weight of lactose monohydrate; 5.0% by weight of croscarmellose sodium; and 1.0% by weight of magnesium stearate.

Another aspect of the invention is a process for the preparation of the wet granulation composition as defined above. The process for the manufacturing of these compositions comprises any method known in the art which comprises a wet granulation step. In an embodiment, optionally in combination with one or more features of the various embodiments described above or below, the process for the preparation of the wet granulation composition as defined above comprises: (a) preparing a granulation dispersion of apixaban by: (i) heating a mixture selected from the group consisting of C₁-C₄ ketone and water; C₁-C₄ alcohol and water; C₁-C₄ ketone and C₁-C₄ alcohol; and C₁-C₄ ketone, C₁-C₄ alcohol, and water at a temperature from 50°C to 70°C; (ii) adding the surfactant and optionally the binder in the solution obtained in step (i); and (iii) adding amorphous apixaban, or alternatively crystalline apixaban, or alternatively a mixture of amorphous and crystalline apixaban in the solution obtained in step (ii) at a temperature from 50°C to 70°C; and (b) applying the granulation dispersion of apixaban obtained in step (a) onto a filler by spray drying; or alternatively (a) preparing a granulation dispersion of apixaban by: (i) heating a mixture selected from the group consisting of C₁-C₄ ketone and water; C₁-C₄ alcohol and water; C₁-C₄ ketone and C₁-C₄ alcohol; and C₁-C₄ ketone, C₁-C₄ alcohol, and water at a temperature from 50°C to 70°C; (ii) adding amorphous apixaban, or alternatively crystalline apixaban, or alternatively a mixture of amorphous and crystalline apixaban in the solution obtained in step (i) at a temperature from 50°C to 70°C; and (iii) adding the surfactant and optionally the binder in the solution obtained in step (ii); and (b) applying the granulation dispersion of apixaban obtained in step (a) onto a filler by spray drying. This process is advantageous because it allows preparing a pharmaceutical composition comprising a therapeutically effective amount of apixaban or a salt thereof together with one or more pharmaceutically acceptable excipients or carriers that has the targeted in vitro dissolution profile.

In an embodiment, optionally in combination with one or more features of the various embodiments described above or below, the process for the preparation of the wet granulation composition is that wherein the apixaban used as starting material is amorphous apixaban. In an alternative embodiment, optionally in combination with one or more features of the various embodiments described above or below, the process for the preparation of the wet granulation composition is that wherein the apixaban used as starting material is crystalline apixaban. In another alternative embodiment, optionally in combination with one or more features of the various embodiments described above or below, the process for the preparation of the wet granulation composition is that wherein the apixaban used as starting material is a mixture of amorphous and crystalline apixaban.

In an embodiment, optionally in combination with one or more features of the various embodiments described above or below, the process for the preparation of the wet granulation composition is that wherein the C₁-C₄ ketone is selected from the group consisting of acetone, propanone and butanone; and the C₁-C₄ alcohol is methanol, ethanol, isopropanol, and butanol; preferably the C₁-C₄ ketone is acetone, and the C₁-C₄ alcohol is isopropanol. In an embodiment, optionally in combination with one or more features of the various embodiments described above or below, the process for the preparation of the wet granulation composition is that wherein the C₁-C₄ ketone is selected from acetone and the C₁-C₄ alcohol is isopropanol.

In an embodiment, optionally in combination with one or more features of the various embodiments described above or below, the process for the preparation of the wet granulation composition is performed by heating a mixture of C₁-C₄ ketone and water. In an embodiment, optionally in combination with one or more features of the various embodiments described above or below, the process for the preparation of the wet granulation composition is that wherein the C₁-C₄ ketone is from 95% to 70% by weight in water.

In an embodiment, the optionally in combination with one or more features of the various embodiments described above or below, the process for the preparation of the wet granulation composition is performed by heating a mixture of C₁-C₄ alcohol and water. In an embodiment, the optionally in combination with one or more features of the various embodiments described above or below, the process for the preparation of the wet granulation composition is that wherein the C₁-C₄ alcohol is from 95% to 70% by weight in water.

In an embodiment, optionally in combination with one or more features of the various embodiments described above or below, the process for the preparation of the wet granulation composition is performed by heating a mixture of C₁-C₄ ketone and C₁-C₄ alcohol. In an embodiment, optionally in combination with one or more features of the various embodiments described above or below, the process for the preparation of the wet granulation composition is that wherein the C₁-C₄ ketone is from 70% to 30% by weight in C₁-C₄ alcohol.

In another alternative embodiment, optionally in combination with one or more features of the various embodiments described above or below, the process for the preparation of the wet granulation composition is performed by heating a mixture of C₁-C₄ ketone, C₁-C₄ alcohol, and water. In an embodiment, optionally in combination with one or more features of the various embodiments described above or below, the process for the preparation of the wet granulation composition is that wherein the weight ratio between the C₁-C₄ ketone, the C₁-C₄ alcohol, and water is comprised 20-1:10-1:1, preferably the weight ratio between the C₁-C₄ ketone, the C₁-C₄ alcohol, and water is 1:1:1. In a particular embodiment, optionally in combination with one or more features of the various embodiments described above or below, the process for the preparation of the wet granulation composition is that wherein step (i) is performed by heating a mixture of C₁-C₄ ketone, C₁-C₄ alcohol, and water, being C₁-C₄ ketone acetone, C₁-C₄ alcohol isopropanol and being the weight ratio between C₁-C₄ ketone, C₁-C₄ alcohol and water of 1:1:1.

In an embodiment, optionally in combination with one or more features of the various embodiments described above or below, the process for the preparation of the wet granulation composition is that wherein the concentration of apixaban in the granulation dispersion is from 1% to 5%; preferably from 1% to 3%. In a particular embodiment, optionally in combination with one or more features of the various embodiments described above or below, the concentration of apixaban in the granulation dispersion is 1.6 %.

In an embodiment, optionally in combination with one or more features of the various embodiments described above or below, the process for the preparation of the wet granulation composition is that wherein step (i) is performed at a temperature from 55°C to 65°C; preferably at a 60°C.

In an embodiment, optionally in combination with one or more features of the various embodiments described above or below, the process for the preparation of the wet granulation composition is that wherein the spray drying of step (b) is performed at a spray rate from 10 to 100 mL/min; at an atomizing air pressure from 0,5 to 2 bar; and at an inlet air flow from 30 to 70 m³/h. Preferably, the spray drying of step (b) is performed at a spray rate of 40 mL/min; at an atomizing air pressure of 1 bar; and at an inlet air flow from 40 to 60 m³/h.

In an embodiment, optionally in combination with one or more features of the various embodiments described above or below, the process for the preparation of the wet granulation composition of the present invention further comprises additional steps (c)-(f) after step (b) comprising: (c) drying the wet granules obtained in step (b); (d) mixing the granules obtained in step (c) with the disintegrant and the lubricant; (e) compressing the mixture obtained in step (d) into tablets; and optionally, (f) film coating the tablets obtained in step (e).

In an embodiment, optionally in combination with one or more features of the various embodiments described above or below, the process for the preparation of the wet granulation composition of the present invention further comprises performing the drying step (c) at a temperature from 45°C to 60°C; preferably from 50°C to 55°C. In an embodiment, optionally in combination with one or more features of the various embodiments described above or below, the process for the preparation of the wet granulation composition of the present invention further comprises performing the drying step (c) until the loss on drying of the obtained granules is equal to or lower than 3%; preferably equal to or lower than 2%.

In an embodiment, optionally in combination with one or more features of the various embodiments described above or below, the process for the preparation of the wet granulation composition of the present invention wherein in step (d) the disintegrant and the lubricant are those disclosed in the present invention. All the embodiments disclosed above for the wet granulation compositions apply also for the process for the preparation of the wet granulation tablets.

In an embodiment, optionally in combination with one or more features of the various embodiments described above or below, the process for the preparation of the wet granulation composition of the present invention further comprises performing the compressing step (e) at a compression force from 3 to 10 kN; particularly at 4.5 kN to obtain tablets having a weight of 100 mg or 200 mg.

All the embodiments disclosed above for the wet granulation compositions of the invention apply also for the preparation process of the wet granulation compositions, preferably for the preparation process of the wet granulation tablets.

The wet granulation composition of the present invention may be defined by its preparation process as defined above and therefore, the wet granulation composition of the present invention obtainable by the process defined above is considered part of the invention. Thus, the wet granulation composition as defined above is obtainable by the process comprising: (a) preparing a granulation dispersion of apixaban by: (i) heating a mixture selected from the group consisting of C₁-C₄ ketone and water; C₁-C₄ alcohol and water; C₁-C₄ ketone and C₁-C₄ alcohol; and C₁-C₄ ketone, C₁-C₄ alcohol, and water at a temperature from 50°C to 70°C; (ii) adding the surfactant and optionally the binder in the solution obtained in step (i); and (iii) adding amorphous apixaban, or alternatively crystalline apixaban, or alternatively a mixture of amorphous and crystalline apixaban in the solution obtained in step (ii) at a temperature from 50°C to 70°C; and (b) applying the granulation of amorphous apixaban obtained in step (a) onto a filler by spray drying; or alternatively (a) preparing a granulation dispersion of apixaban by:
(i) heating a mixture selected from the group consisting of C₁-C₄ ketone and water; C₁-C₄ alcohol and water; C₁-C₄ ketone and C₁-C₄ alcohol; and C₁-C₄ ketone, C₁-C₄ alcohol, and water at a temperature from 50°C to 70°C;
(ii) adding amorphous apixaban, or alternatively crystalline apixaban, or alternatively a mixture of amorphous and crystalline apixaban in the solution obtained in step (i) at a temperature from 50°C to 70°C; and (iii) adding the surfactant and optionally the binder in the solution obtained in step (ii); and (b) applying the granulation dispersion of apixaban obtained in step (a) onto a filler by spray drying. As it is illustrated in the examples, this process is advantageous because it allows preparing a pharmaceutical composition comprising a therapeutically effective amount of apixaban or a salt thereof having the targeted in vitro dissolution profile.

In an embodiment, optionally in combination with one or more features of the various embodiments described above or below, the wet granulation composition as defined above is obtainable by the process comprising: (a) preparing a granulation dispersion of apixaban by: (i) heating a mixture of C₁-C₄ ketone, C₁-C₄ alcohol, and water at a temperature from 50 to 70°C; (ii) adding the surfactant and optionally the binder in the solution obtained in step (i); and (iii) adding amorphous apixaban, or alternatively crystalline apixaban, or alternatively a mixture of amorphous and crystalline apixaban in the solution obtained in step (ii) at a temperature from 50°C to 70°C; and (b) applying the granulation dispersion of apixaban obtained in step (a) onto a filler by spray drying; or alternatively (a) preparing a granulation dispersion of apixaban by: (i) heating a mixture of C₁-C₄ ketone, C₁-C₄ alcohol, and water at a temperature from 50 to 70°C; (ii) adding amorphous apixaban, or alternatively crystalline apixaban, or alternatively a mixture of amorphous and crystalline apixaban in the solution obtained in step (i) at a temperature from 50°C to 70°C; and (iii) adding the surfactant and optionally the binder in the solution obtained in step (ii); and (b) applying the granulation dispersion of apixaban obtained in step (a) onto a filler by spray drying; (c) drying the wet granules obtained in step (b); (d) mixing the granules obtained in step (c) with the disintegrant and the lubricant; (e) compressing the mixture obtained in step (d) into tablets; and optionally, (f) film coating the tablets obtained in step (e).

All the embodiments disclosed above for the process of the preparation of the wet granulation tablets of the invention apply also for the wet granulation tablets obtainable by this process.

In an alternative embodiment, optionally in combination with one or more features of the various embodiments described above or below, the composition of the invention is a hot-melt extruded tablet.

The term "hot-melt extruded" refers to a composition whose components may be hot-melt extruded. Particularly, a hot-melt extrudate is obtained from a mixture of the active ingredient and the pharmaceutically acceptable excipients or carriers by hot-melt extrusion which is later milled and compressed into tablets together with appropriate excipients and carriers.

The term "extrudate" refers to a mixture of apixaban, at least one hot-melt extrudable polymer and at least one hot-melt extrudable plasticizer, which are subjected to a hot-melt extrusion. For the purposes of the invention an "extrudate" is the mixture obtained after carrying out steps (i) and (ii) of the process of the invention. Particularly, the extrudate is the mixture obtained by the process which comprises (i) mixing amorphous apixaban, or alternatively crystalline apixaban, or alternatively a mixture of amorphous and crystalline apixaban with the hot-melt extrudable polymer and the hot-melt extrudable plasticizer; and (ii) extruding the mixture obtained in step (i) by hot melt extrusion.

The term "hot-melt extrudable polymer" refers to a polymer that is sufficiently rigid at room temperature and pressure but is capable of being deformed or capable of forming a semi-liquid state under elevated heat and/or pressure. Appropriate hot-melt extrudable polymer for the present invention includes, but is not limited to, cellulose derivates, hydroxypropyl cellulose, hydroxypropylmethyl cellulose, polyvinyl caprolactam-polyvinyl acetate-polyethylen glycol graft copolymer, polyvinylpyrolidone, polyvinyl alcohol-polyethylen glycol graft copolymer, poly(methacrylate) derivative, poly(ethylene-co-vinyl acetate), poly(ethylene), poly(vinyl acetate-co-methacrylic acid), epoxy resins, caprolactones, poly(ethylene oxide), vinylpyrrolidone-vinyl acetate copolymers and mixture thereof. Preferably the hot-melt extrudable polymer is selected from vinylpyrrolidone-vinyl acetate copolymer, polyvinyl caprolactam-polyvinyl acetate-polyethylen glycol graft copolymer, polyvinylpyrolidone, polyvinyl alcohol-polyethylen glycol graft copolymer, and mixtures thereof.

The term "hot-melt extrudable plasticizer" refers to any compound capable of plasticizing hot-melt extruded polymer, and also generally reduces the viscosity of a polymer melt thereby allowing for lower processing temperature and extruder torque during hot-melt extrusion. Appropriate hot-melt extrudable plasticizer for the present invention includes, but is not limited to, low molecular weight polymers, oligomers, copolymers, oils, small organic molecules, low molecular weight polyols having aliphatic hydroxyls, ester-type plasticizers, glycol ethers, poly(propylene glycol), multi-block polymers, single block polymers, low molecular weight poly(ethylene oxide) (average molecular weight less than about 500,000) and poly(ethylene glycol). Examples of suitable hot-melt extrudable plasticizer can be ethylene glycol, propylene glycol, 1,2-butylene glycol, 2,3-butylene glycol, styrene glycol, diethylene glycol, triethylene glycol, tetraethylene glycol and other poly(ethylene glycol) compounds, polyethylene oxide-polypropylen oxide copolymer, monopropylene glycol monoisopropyl ether, propylene glycol monoethyl ether, ethylene glycol monoethyl ether, diethylene glycol monoethyl ether, sorbitol lactate, ethyl lactate, butyl lactate, ethyl glycolate, triethyl citrate, acetyl triethyl citrate, tributyl citrate and allyl glycolate. Preferably, the hot-melt extrudable plasticizer is selected from the group of poly(ethylene glycol), polyethylene oxide-polypropylen oxide copolymer and mixtures thereof; more preferably poly (ethylene glycol) such as for example PEG 4000 and polyethylene oxide-polypropylen oxide copolymer such as for example Poloxamer 188.

In an embodiment, optionally in combination with one or more features of the various embodiments described above or below, the composition of the invention is a hot-melt extruded tablet and the composition comprises: from 1-20% by weight of amorphous apixaban; from 10-90% by weight of hot-melt extrudable polymer; from 1-30% by weight of hot-melt extrudable plasticizer.

In an embodiment, optionally in combination with one or more features of the various embodiments described above or below, the composition of the invention is a hot-melt extruded tablet and the composition further comprises extra-granular excipients. In an embodiment, optionally in combination with one or more features of the various embodiments described above or below, the composition of the invention is a hot-melt extruded tablet and the composition further comprises extra-granular excipients selected from the group consisting of one or more fillers, disintegrants, lubricants and mixtures thereof.

All the embodiments disclosed above for fillers, disintegrants, lubricants appropriate for the wet granulation compositions of the invention apply also for the hot-melt extruded tablets.

In an embodiment, optionally in combination with one or more features of the various embodiments described above or below, the composition of the invention is a hot-melt extruded tablet and the composition comprises: from 1-10% by weight of amorphous apixaban; from 10-90% by weight of hot-melt extrudable polymer; from 1-30% by weight of hot-melt extrudable plasticizer; from 10-80% by weight of filler; from 1-20% by weight of disintegrant; and from 1-5% by weight of lubricant; being the sum of the ingredients 100%.

In an embodiment, optionally in combination with one or more features of the various embodiments described above or below, the composition of the invention is a hot-melt extruded tablet and the composition comprises: from 1-10% by weight of amorphous apixaban; from 10-90% by weight of vinylpyrrolidone-vinyl acetate copolymers; from 1-30% by weight of poly(ethylene glycol); from 10-80% by weight of lactose monohydrate; from 1-20% by weight of croscarmellose sodium; and from 1-5% by weight of magnesium stearate; being the sum of the ingredients 100%.

In a particular embodiment, optionally in combination with one or more features of the various embodiments described above or below, the composition of the invention is a hot-melt extruded tablet and the composition comprises: 2.5% by weight of amorphous apixaban; 20.0% by weight of vinylpyrrolidone-vinyl acetate copolymers; 2.5% by weight of poly(ethylene glycol); 69.5% by weight of lactose monohydrate; 4.0% by weight of croscarmellose sodium; and 1.5% by weight of magnesium stearate.

In another particular embodiment, optionally in combination with one or more features of the various embodiments described above or below, the composition of the invention is a hot-melt extruded tablet and the composition comprises: 2.5% by weight of amorphous apixaban; 25.6% by weight of vinylpyrrolidone-vinyl acetate copolymers; 3.13% by weight of poly(ethylene glycol); 63.25% by weight of lactose monohydrate; 4.0% by weight of croscarmellose sodium; and 1.5% by weight of magnesium stearate.

In a further particular embodiment, optionally in combination with one or more features of the various embodiments described above or below, the composition of the invention is a hot-melt extruded tablet and the composition comprises: 2.5% by weight of amorphous apixaban; 42.5% by weight of vinylpyrrolidone-vinyl acetate copolymers; 5% by weight of poly(ethylene glycol); 44.5% by weight of lactose monohydrate; 4.0% by weight of croscarmellose sodium; and 1.5% by weight of magnesium stearate.

In a further particular embodiment, optionally in combination with one or more features of the various embodiments described above or below, the composition of the invention is a hot-melt extruded tablet and the composition comprises: 2.5% by weight of amorphous apixaban; 25.62% by weight of vinylpyrrolidone-vinyl acetate copolymers; 3.13% by weight of polyoxyethylene/polyoxypropylene copolymer; 63.25% by weight of lactose monohydrate; 4.0% by weight of croscarmellose sodium; and 1.5% by weight of magnesium stearate.

Another aspect of the invention is a process for the preparation of the hot-melt extruded composition. The process for the manufacturing of these compositions comprises any method known in the art which comprises a hot-melt extrusion process. In an embodiment, optionally in combination with one or more features of the various embodiments described above or below, the process for the preparation of the hot-melt extruded tablet as defined above comprises: (i) mixing amorphous apixaban, or alternatively crystalline apixaban, or alternatively a mixture of amorphous and crystalline apixaban with the hot-melt extrudable polymer and the hot-melt extrudable plasticizer; (ii) extruding the mixture obtained in step (i) by hot melt extrusion to obtain an extrudate; (iii) milling the extrudate obtained in step (ii); (iv) mixing the filler, the disintegrant and the lubricant to obtain a mixture A; (v) mixing the mixture A obtained in step (iv) with the extrudate obtained in step (ii); (vi) compressing the mixture obtained in step (v) into tablets; and optionally, (vii) film coating the tablets obtained in step (vi) is also part of the invention. As it is illustrated in the examples, this process is advantageous because it allows preparing a pharmaceutical composition comprising a therapeutically effective amount of apixaban or a salt thereof having the targeted in vitro dissolution profile.

In an embodiment, optionally in combination with one or more features of the various embodiments described above or below, the process for the preparation of the hot-melt extruded composition is that wherein step (ii) is performed at a melting temperature from 140°C to 240°C; preferably from 160°C to 220°C.

In an embodiment, optionally in combination with one or more features of the various embodiments described above or below, the process for the preparation of the hot-melt extruded composition is that wherein step (ii) is performed at a feeding rate from 0.05 kg/h to 1.5 kg/h; preferably from 0.1 kg/h to 0.4 kg/h. Particularly, the feeding rate is 0.3 kg/h.

In an embodiment, optionally in combination with one or more features of the various embodiments described above or below, the process for the preparation of the hot-melt extruded composition is that wherein step (ii) is performed at a feeding temperature from 15°C to 50°C; preferably from 20°C to 40°C.

In an embodiment, optionally in combination with one or more features of the various embodiments described above or below, the process for the preparation of the hot-melt extruded composition is that wherein step (ii) is performed at a pressure at the end of the die from 0.5 bar to 2 bars; preferably 1 bar.

In an embodiment, optionally in combination with one or more features of the various embodiments described above or below, the process for the preparation of the hot-melt extruded composition further comprises performing the compressing step (vi) at a compression force from 3 kN to 15 kN; particularly at 8.0 kN to obtain tablets having a weight of 100 mg or 200 mg.

All the embodiments disclosed above for the hot-melt extruded compositions of the invention apply also for the preparation process of the hot-melt extruded compositions.

The hot-melt extruded composition of the present invention may be defined by its preparation process as defined above and therefore, the hot-melt extruded tablet of the present invention obtainable by the process defined above is part of the invention. Thus, the hot-melt extruded tablet as defined above is obtainable by the process comprising: (i) mixing amorphous apixaban, or alternatively crystalline apixaban, or alternatively a mixture of amorphous and crystalline apixaban with the hot-melt extrudable polymer and the hot-melt extrudable plasticizer; (ii) extruding the mixture obtained in step (i) by hot melt extrusion to obtain an extrudate; (iii) milling the extrudate obtained in step (ii); (iv) mixing the filler, the disintegrant and the lubricant to obtain a mixture A; (v) mixing the mixture A obtained in step (iv) with the extrudate obtained in step (ii); (vi) compressing the mixture obtained in step (v) into tablets; and optionally, (vii) film coating the tablets obtained in step (vi) is also part of the invention.

All the embodiments disclosed above for the process of the preparation of the hot-melt extruded tablets of the invention apply also for the hot-melt extruded tablet obtainable by this process.

In an embodiment, optionally in combination with one or more features of the various embodiments described above or below, the pharmaceutical compositions as defined above of the present invention further comprise an outer coating layer; preferably an outer film coating layer. This outer coating layer comprises at least an appropriate polymer. Appropriate polymers for preparing the outer film coating layer include, but are not limited to, hydroxylpropylmethyl cellulose, polyvinyl alcohol, copovidone, polyvinyl alcohol-polyethylen glycol copolymer, and mixtures thereof. The above mentioned polymer of the additional outer film coating can be accompanied by plasticizers such as triethylcitrate (TEC), polyethylene glycol (PEG), cetyl and stearyl alcohol; surface-active agents such as sodium lauryl sulphate, polysorbate and poloxamer; pigments such as titanium dioxide, iron sesquioxide; lubricants such as talc, magnesium stearate or glyceril monostearate, and mixtures thereof. This additional outer film coating can be prepared by any film coating method known in the state of the art.

As mentioned above, it is also part of the invention the pharmaceutical composition comprising a therapeutically effective amount of apixaban as defined above for use as anticoagulant, in particular in the treatment of a venous thromboembolic disease or condition. This aspect could be also formulated as the use of the pharmaceutical composition comprising a therapeutically effective amount of apixaban as defined above for the preparation of a medicament for the prophylaxis and/or treatment of a venous thromboembolic disease or condition. It also relates to a method for the prophylaxis and/or treatment of a mammal suffering or is susceptible to suffer from a venous thromboembolic disease or condition; the method comprises administering to said mammal an effective amount of the pharmaceutical composition comprising a therapeutically effective amount of apixaban as defined above.

Throughout the description and claims the word "comprise" and variations of the word, are not intended to exclude other technical features, additives, components, or steps. Furthermore, the word "comprise" encompasses the case of "consisting of'. Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### EXAMPLES

### General consideration

Determination of particle size distribution of the apixaban used as a starting material for the preparation of the pharmaceutical compositions of the invention was performed by the following methods:
1. laser diffraction spectroscopy using a Malvern Mastersizer 2000 Hydro 2000S particle size analyser with cyclohexane and span 85 as sample dispersant, stir speed of 2500 rpm, sample concentration 100 mg/10 mL and measurement time of 12 seconds.
2. G3 Morphologi (Malvern), Sample volume: 5 mm3, automatic dispersion (Sample Dispersion Unit): Pressure 3 bra, Injection Time 60 seconds, Settling Time 120 seconds; Measurement condition: Diascopic Light, Automated light calibration, Optic magnification 20x. The images of aggregate or small (below 50 pixels) particles were filtered out.
3. Raman spectra using Nicolet Almega XR Raman Spectrometer. Instrumental parameters are: excitation wavelength 780 nm; spectrograph aperture 100 µm pinhole; objective magnification 50x long distance; exposure time 1 second; step 2,5 in x direction and 2,5 µm in y direction. Prior to the collection of spectral data the tablet was trimmed to achieve a flat surface.

### 1. Compositions of apixaban of the present invention

### 1.1. Compositions

The following examples of Tables 1-2 illustrate the compositions in accordance with the present invention, which comprises apixaban being amorphous apixaban and exhibit the target dissolution profile.

Table 1 illustrates the wet granulation tablets of the present invention, where the amount of the ingredients is expressed in % per tablet (Examples 1-3) and grams (Example 4) per tablet.

**Table 1**

| **Ingredients** | | **Example 1 (%)** | **Example 2 (%)** | **Example 3 (%)** | **Example 4 (g)** |
|---|---|---|---|---|---|
| | Amorphous Apixaban (Active ingredient) | 2.50 | 2.50 | 2.50 | 25.00 |
| | Lactose monohydrate (Filler) | 71.50 | 80.25 | 80.25 | 802.50 |
| Intra-Granular | Sodium laurilsulfate (Surfactant) | - | 1.25 | 1.25 | 12.50 |
| | Polyvinylpyrrolidone k25 (binder) | 10.00 | - | 10.00 | 100.00 |
| | Poloxamer 188⁽¹⁾ (Surfactant) | 10.00 | 10.00 | - | - |
| Extra-granular | Croscarmellose sodium (Disintegrant) | 5.00 | 5.00 | 5.00 | 50.00 |
| | Magnesium stearate (Lubricant) | 1.00 | 1.00 | 1.00 | 10.00 |
| Total weight | | 100% | 100% | 100% | 1000 g |

| | | | | | |
|---|---|---|---|---|---|
| ⁽¹⁾ Poloxamer 188 is a polyoxyethylene-polyoxypropylene block copolymer which is marketed with the tradename Pluronic^{®} F-68 | | | | | |

Tables 2 and 3 illustrate the hot-melt extruded tablets of the present invention, where the amount of each ingredient is expressed in percentage (%) by weight and also in weight (mg) per tablet.

**Table 2**

| **Ingredients** | | **Example 5** | | **Example 6** | | **Example 7** | |
|---|---|---|---|---|---|---|---|
| | | **(%)** | **mg/ tablet** | **(%)** | **mg/ tablet** | **(%)** | **mg/ tablet** |
| Hot-melt extrudable ingredients | Apixaban amorphous (Active ingredient) | 2.5 | 5.0 | 2.5 | 5 | 2.5 | 5 |
| | Kollidon VA64⁽¹⁾ (Hot-melt extrudable polymer) | 20.0 | 40.0 | 25.62 | 51.25 | 42.5 | 85 |
| | Polyethyleneglycol (PEG) 4000 (Hot-melt extrudable plasticizer) | 2.5 | 5.0 | 3.13 | 6.25 | 5 | 10 |
| Extra-granular | Lactose monohydrate (Filler) | 69.5 | 139.0 | 63.25 | 126.5 | 44.5 | 89 |
| | Croscarmellose sodium (Disintegrant) | 4.0 | 8.0 | 4.0 | 8.0 | 4.0 | 8.0 |
| | Magnesium stearate (Lubricant) | 1.5 | 3.0 | 1.5 | 3.0 | 1.5 | 3.0 |
| Total weight | | 100 | 200 | 100 | 200 | 100 | 200 |

| | | | | | | | |
|---|---|---|---|---|---|---|---|
| (1) Kollidon VA64 is a vinylpyrrolidone-vinyl acetate copolymers which is marketed by BASF | | | | | | | |

**Table 3**

| **Ingredients** | | | **Example 8** | |
|---|---|---|---|---|
| | | | **(%)** | **mg/tablet** |
| Hot-melt extrudable ingredients | Apixaban amorphous | Active ingredient | 2.5 | 5.0 |
| | Kollidon VA64⁽¹⁾ | Hot-melt extrudable polymer | 25.62 | 51.25 |
| | Poloxamer 188⁽²⁾ | Hot-melt extrudable plasticizer | 3.13 | 6.25 |
| Extra-granular | Lactose monohydrate | Filler | 63.25 | 126.5 |
| | Croscarmellose sodium | Disintegrant | 4 | 8.0 |
| | Magnesium stearate | Lubricant | 1.5 | 3.0 |
| Total weight | | | 100 | 200 |

| | | | | |
|---|---|---|---|---|
| (1) Kollidon VA64 is a vinylpyrrolidone-vinyl acetate copolymers which is marketed by BASF (2) Poloxamer 188 is a polyoxyethylene/polyoxypropylene copolymer which is marketed by BASF | | | | |

### 1.2. Preparation process

### Wet granulation process

The compositions of Examples 1-4 of the present invention disclosed in Table 1 were prepared following the process which comprises a wet granulation process comprising the following steps (a)-(f):
(a) preparing a granulation dispersion of apixaban which comprises: (i) mixing acetone (41.6 mL), isopropanol (41.6 mL), and water (41.6 mL) to obtain 125 mL of the solution and heating the resulting solution at a 60°C; (ii) to the solution obtained in step (i), the intra-granular surfactant and the intra-granular binder were added and the resulting mixture was continuously stirred until total dissolution and heated at a 60°C; and (iii) adding crystalline apixaban (D90= 178.8 µm) to the solution obtained in step (ii) under stirring until an opalescent solution was obtained.
(b) applying the granulation dispersion of apixaban obtained in step (a) onto the intra-granular filler by spray drying;
(c) drying the wet granules obtained in step (b);
(d) mixing the granules obtained in step (c) with the extra-granular excipients;
(e) compressing the mixture obtained in step (d) into tablets, and optionally,
(f) film coating the tablets obtained in step (e).

The composition of Example 4 of the present invention disclosed in Table 1 was prepared following the process as defined for examples 1-3 using the corresponding amount of ingredients and 1250 mL of a mixture of 1:1:1 acetone/isopropanol/water, which is prepared mixing 416.67 mL of each component of the mixture.

### Hot melt process

The composition of Examples 5-7 of the present invention disclosed in Table 2 was prepared following the process which comprises a hot-melt extrusion process which comprises steps (i)-(vii) as defined below:
(i) weighing and mixing crystalline apixaban (D90= 17µm), kollidon VA64 and PEG 4000;
(ii) extruding the mixture obtained in step (i) by hot-melt extrusion using a thermo Fisher Tween Screw Extruder Pharma 11 at a feeding rate of 0.3kg/h, a screws speed of 150 RPM, at a pressure at the end of the die of 1 bar, at a feeding zone temperature from 20°C to 40°C; at a melting zone temperature from 160°C to 200°C; and at a die temperature from 160°C to 200°C.
(iii) milling the extrudate obtained in step (ii);
(iv) weighing and mixing the lactose monohydrate, croscarmellose sodium and magnesium stearate to obtain a mixture A;
(v) mixing the mixture A obtained in step (iv) with the extrudate obtained in step (ii);
(vi) compressing the mixture obtained in step (v) into tablets using a single punch tablet press machine (Korsch XP1) at a compression force of 8,0kN obtaining a tablet weight of 200mg;
(vii) film coating the tablets obtained in step (vi).

The composition of Example 8 of the present invention disclosed in Table 3 was prepared following the process as defined for examples 5-7 using the corresponding amount of ingredients and using poloxamer 188 instead of PEG 4000.

### 2. Comparative compositions

### 2.1. Compositions

The following Examples of Table 4 illustrate the compositions of the comparative examples which are outside the scope of the present invention, where the amount of each ingredient is expressed in weight per cent (%).

**Table 4**

| **Ingredients** | | | **Comparative Example 1 Eliquis®**^{(**1**)} | **Comparative Example 2⁽²⁾** |
|---|---|---|---|---|
| Intra-granular | Apixaban | Active ingredient | 2.5 | 2.50 |
| | Lactose monohydrate | filler | 50.25 | 50.00 |
| | Sodium laurilsulfate | surfactant | 1.00 | 1.00 |
| | Polyvinylpyrrolidone k25 | binder | 1.25 | 4.00 |
| | Croscarmellose sodium | disintegrant | 4.00 | 2.00 |
| | Microcrystalline cellulose | filler | 41.00 | 37.75 |
| Extra-granular | Croscarmellose sodium | disintegrant | - | 2.00 |
| | Magnesium stearate | lubricant | - | 0.75 |

| | | | | |
|---|---|---|---|---|
| ⁽¹⁾ Comparative Example 1 corresponds to the batch number 4H67975A of Eliquis® that contain crystalline apixaban having a D₁₀₀ of less than 10 µm measured by Raman spectra. ⁽²⁾ Comparative Example 2 corresponds to Example 3 disclosed in the PCT patent application WO2015121472 of the state of the art. The apixaban used as a starting material repeating Example 3 of the PCT patent application WO2015121472 is crystalline apixaban having a D₉₀ = 50 µm. | | | | |

### 3. Dissolution profile

The target dissolution profile requires that at least 30% of apixaban is dissolved after 5 minutes and at least 60% of apixaban is dissolved after 10 minutes when measured in phosphate buffer at pH 6.8 containing 0.05 % weight/volume of sodium lauryl sulfate.

### 3.1. Comparative dissolution profile between Eliquis and the compositions of the present invention

The dissolution test was carried out to a solution of 900 mL at 37°C and at 75rpm measured in phosphate buffer at pH 6.8 containing 0.05% weight/volume of sodium lauryl sulfate using a Hanson Research as a dissolution apparatus according to the conditions described in the USP30 Pharmacopoeia.

### Conditions of the dissolution bath

- Paddle speed: 75 rpm
- Temperature of dissolution medium: 37 °C ± 0.5 °C
- Buffered step: pH 6.8
- Vessel volume: 900 mL

### Conditions of the chromatographic analysis

- Flux: 1,0 mL/min
- Column: Waters X-terra RP18 5um 150x3,9
- Phases: Acetonitrile (CAN): 0,1% TFA (Trifluoroacetic acid)

| | **Time** | **Flow** | **% CAN** | **% TFA** | **Curve** |
|---|---|---|---|---|---|
| 1 | 0.01 | 1.00 | 40.0 | 60.0 | 6 |
| 2 | 5.50 | 1.00 | 80.0 | 20.0 | 6 |
| 3 | 5.60 | 1.00 | 40.0 | 60.0 | 6 |
| 4 | 9.00 | 1.00 | 40.0 | 60.0 | 6 |

- Column temperature: 40 °C
- Autosampler temperature 20 °C
- Injection volume: 50µL
- Excitation wavelength: 270 nm

Table 5 shows the dissolution profile at pH 6.8 of Eliquis (comparative Example 1 having crystalline apixaban), the comparative Example 2 (having crystalline apixaban) and the wet granulation compositions of the invention (Examples 1-4, having amorphous apixaban).

**Table 5**

| **Time (hours)** | **Dissolved apixaban (%)** | | | | | |
|---|---|---|---|---|---|---|
| | **Comparative Examples** | | **Examples** | | | |
| | **1** | **2** | **1** | **2** | **3** | **4** |
| **5** | **44.4** | **36.5** | **34.4** | **32.8** | **45.4** | **44.1** |
| **10** | **83.7** | **45.5** | **64.7** | **65.1** | **95.2** | **74.1** |
| 15 | 91.2 | 50.9 | 85.9 | 85.9 | 94.4 | 93 |
| 20 | 94.1 | 54.6 | 98.2 | 96.5 | 95.2 | 99 |
| 30 | 97.4 | 59.2 | 102.3 | 100.1 | 95.2 | 99.4 |
| 40 | 98.8 | 64.2 | 101.9 | 100.4 | 95.2 | 99.4 |

Table 6 shows the dissolution profile at pH 6.8 of Eliquis (comparative Example 1, having crystalline apixaban), the comparative Example 2 (having crystalline apixaban) and the hot-melt extruded compositions of the invention (Examples 6 and 8, having amorphous apixaban).

**Table 6**

| **Time (hours)** | **Dissolved apixaban (%)** | | | |
|---|---|---|---|---|
| | **Eliquis Comparative Ex. 1** | **Comparative Ex. 2** | **Example 6** | **Example 8** |
| **5** | **44.4** | **36.5** | **65.8** | **64** |
| **10** | **83.7** | **45.5** | **88.7** | **82.9** |
| 15 | 91.2 | 50.9 | 94.4 | 89.3 |
| 20 | 94.1 | 54.6 | 95.8 | 92.7 |
| 30 | 97.4 | 59.2 | 96.3 | 94 |
| 40 | 98.8 | 64.2 | 96.7 | 95.2 |

The dissolution profile results in Tables 5-6 show that only the compositions of the invention have the required dissolution profile at pH 6.8. Furthermore, these tables also show that the compositions of the invention have a comparable dissolution profile as Eliquis.

### 4. X-ray Powder Diffraction (XRPD)

XRPD is a powerful and widely-used tool for crystalline state evaluation. Diffraction patterns of the compositions were determined using a PANalytical X'Pert PRO MPD diffractometer in Bragg-Brentano geometry, X'Celerator RTMS Detector, with a Cu line as the source of radiation (WL1 = 1.5406 A, WL2 = 1.54439 A), and standard runs using a 45 kV voltage, a 40 mA current and a scanning rate of 0.01 °/min over a 2 θ range of 5° to 40°.

### PRIOR ART REFERENCE MENTIONED IN THE APPLICATION

1. US200726082
2. WO2015121472

## Claims

1. A pharmaceutical composition comprising a therapeutically effective amount of apixaban or a salt thereof together with one or more pharmaceutically acceptable excipients or carriers, wherein apixaban is amorphous and the composition exhibits an in vitro dissolution profile measured in phosphate buffer at pH 6.8 containing 0.05% weight/volume of sodium lauryl sulfate according to which:
at least 30% of apixaban is dissolved after 5 minutes and
at least 60% of apixaban is dissolved after 10 minutes.

2. The composition according to claim 1, wherein the composition exhibits an in vitro dissolution profile measured in phosphate buffer at pH 6.8 containing 0.05% weight/volume of sodium lauryl sulfate according to which:
at least 40% of apixaban is dissolved after 5 minutes and
at least 80% of apixaban is dissolved after 10 minutes.

3. The composition according to any of the claims 1-2, wherein the composition is a wet granulation tablet or a hot-melt extruded tablet.

4. The composition according to claim 3, wherein the composition is a wet granulation tablet and comprises:
from 1-10% by weight of amorphous apixaban;
from 1-12% by weight of surfactant;
from 0-20% by weight of binder;
from 35-95% by weight of filler;
from 1-20% by weight of disintegrant; and
from 1-5% by weight of lubricant;
being the sum of the ingredients 100%.

5. The composition according to claim 3, wherein the composition is a hot-melt extruded tablet and comprises:
from 1-10% by weight of amorphous apixaban;
from 10-90% by weight of hot-melt extrudable polymer;
from 1-30% by weight of hot-melt extrudable plasticizer;
from 10-80% by weight of filler;
from 1-20% by weight of disintegrant; and
from 1-5% by weight of lubricant;
being the sum of the ingredients 100%.

6. The composition as defined in any of the claims 4-5, wherein the filler is lactose monohydrate.

7. A process for the preparation of a composition as defined in any of the claims 1-4 and 6, which comprises:
(a) preparing a granulation dispersion of apixaban by:
(i) heating a mixture selected from the group consisting of C₁-C₄ ketone and water; C₁-C₄ alcohol and water; C₁-C₄ ketone and C₁-C₄ alcohol; and C₁-C₄ ketone, C₁-C₄ alcohol, and water at a temperature from 50 to 70°C;
(ii) adding the surfactant and optionally the binder in the solution obtained in step (i); and
(iii) adding amorphous apixaban, or alternatively crystalline apixaban, or alternatively a mixture of amorphous and crystalline apixaban in the solution obtained in step (ii) at a temperature from 50°C to 70°C; and
(b) applying the granulation dispersion of apixaban obtained in step (a) onto a filler by spray drying; or alternatively.
(a) preparing a granulation dispersion of apixaban by:
(i) heating a mixture selected from the group consisting of C₁-C₄ ketone and water; C₁-C₄ alcohol and water; C₁-C₄ ketone and C₁-C₄ alcohol; and C₁-C₄ ketone, C₁-C₄ alcohol, and water at a temperature from 50°C to 70°C;
(ii) adding amorphous apixaban, or alternatively crystalline apixaban, or alternatively a mixture of amorphous and crystalline apixaban in the solution obtained in step (i) at a temperature from 50°C to 70°C; and
(iii) adding the surfactant and optionally the binder in the solution obtained in step (ii); and
(b) applying the granulation dispersion of apixaban obtained in step (a) onto a filler by spray drying.

8. The process according to claim 7, wherein step (i) of the process is performed by heating a mixture of C₁-C₄ ketone, C₁-C₄ alcohol, and water, being C₁-C₄ ketone acetone, C₁-C₄ alcohol isopropanol and being the weight ratio between C₁-C₄ ketone, C₁-C₄ alcohol and water of 1:1:1.

9. The process according to any of the claims 7-8, wherein the concentration of apixaban in the granulation dispersion is from 1% to 5%.

10. The process according to any of the claims 7-9, further comprises additional steps (c)-(f) after step (b):
(c) drying the wet granules obtained in step (b);
(d) mixing the granules obtained in step (c) with the disintegrant and the lubricant;
(e) compressing the mixture obtained in step (d) into tablets; and optionally,
(f) film coating the tablets obtained in step (e).

11. A process for the preparation of a composition as defined in any of the claims 1-3, 5 and 6 which comprises:
(i) mixing amorphous apixaban, or alternatively crystalline apixaban, or alternatively a mixture of amorphous and crystalline apixaban with the hot-melt extrudable polymer and the hot-melt extrudable plasticizer;
(ii) extruding the mixture obtained in step (i) by hot melt extrusion to obtain an extrudate;
(iii) milling the extrudate obtained in step (ii);
(iv) mixing the filler, the disintegrant and the lubricant to obtain a mixture A;
(v) mixing the mixture A obtained in step (iv) with the extrudate obtained in step (ii);
(vi) compressing the mixture obtained in step (v) into tablets; and optionally,
(vii) film coating the tablets obtained in step (vi).

12. The process according to claim 11, wherein the hot-melt extrudable polymer is a vinylpyrrolidone-vinyl acetate copolymer and the hot-melt extrudable plasticizer is polyethylene glycol, or alternatively the hot-melt extrudable polymer is a vinylpyrrolidone-vinyl acetate copolymer and the hot-melt extrudable plasticizer is polyethylene oxide-polypropylen oxide copolymer.

13. The process according to any of the claims 11-12, wherein step (ii) is performed at a melting temperature from 140°C to 240°C and at a feeding rate from 0.05 kg/h to 1.5 kg/h.

14. The process according to claim 13, wherein the feeding temperature is from 15°C to 50°C and the pressure at the end of the die is from 0.5 bar to 2 bars.

15. A pharmaceutical composition as defined in any of the claims 1-6, for use in the treatment of a venous thromboembolic disease or disorder.
